# FASCICULE DE BREVET EUROPEEN

(11) **EP 1 453 889 B1**
(45) Date de publication et mention de la délivrance du brevet: **13.08.2008**
(21) Numéro de dépôt: 02801113.8
(22) Date de dépôt: 10.12.2002
(51) Int. Cl.: C08J 3/075, C08J 3/07

(54) **PARTICULES DE STRUCTURE INTERNE ORGANISEE DISPERSEES DANS UNE PHASE AQUEUSE, PREPARATION ET UTILISATIONS.**
WÄSSRIGE DISPERSIONEN VON IONISCHEN POLYMERPARTIKELN MIT INTERNER MESOPHASE, IHRE HERSTELLLUNG UND VERWENDUNG
PARTICLES HAVING AN ORGANISED INTERNAL STRUCTURE WHICH ARE DISPERSED IN AN AQUEOUS PHASE, THE PREPARATION THEREOF AND USE OF SAME

(30) Priorité: 12.12.2001 US 340381 P
(43) Date de publication de la demande: 08.09.2004
(73) Titulaire: RHODIA CHIMIE, 92512 Boulogne Billancourt Cedex (FR)
(72) Inventeur: BAVOUZET, Bruno, F-75010 Paris (FR); BEYERMANN, Jochen, 81379 Munich (DE); HERVE, Pascal, West Windsor, NJ 08550 (US)
(74) Mandataire: Boittiaux, Vincent
(86) Numéro de dépôt international: PCT/FR2002/004275
(87) Numéro de publication internationale: WO 2003/050166

(56) Documents cités:
- WO-A-95/04774
- WO-A-02/077075

## Description

La présente invention a pour objet des particules en suspension dans une phase aqueuse, dont la phase interne est organisée en mésophase, et comprenant un polymère hydrosoluble chargé ioniquement, un tensioactif de charge opposée à celle du polymère précité, et un copolymère à blocs hydrosoluble.

Les mélanges aqueux comprenant un polymère et un tensioactif de charges ioniques opposées mènent généralement à une séparation macroscopique de phases, dont l'une est concentrée en tensioactif et polymère, l'autre diluée.

La phase concentrée peut dans certaines conditions être une phase structurée et présenter un ordre lamellaire, cubique, hexagonal, etc.

Récemment, on a pu, à partir d'une telle phase organisée, obtenir par un procédé faisant intervenir un solvant, une redispersion aqueuse de particules de taille nanométrique, comprenant une phase organisée de tensioactif et de polymère.

Cependant, ce type de procédé présente de nombreux inconvénients. Tout d'abord, le procédé est complexe, nécessite l'emploi d'un solvant organique. Par ailleurs, la concentration en particules en suspension que l'on peut atteindre en mettant en oeuvre de tels procédés reste trop faible.

L'un des objectifs de la présente invention est de proposer des particules de structure interne organisée et comprenant un polymère chargé et un tensioactif de charge opposée, qui soient stables en solution aqueuse, et dont la concentration soit plus élevée que celles atteintes par les procédés classiques.

Un autre objectif de la présente invention repose dans la mise au point d'un procédé simple, ne mettant pas en oeuvre de composés qui puissent poser des problèmes de toxicité ou d'écotoxicité.

Ces buts et d'autres sont atteints par la présente invention qui a donc pour premier objet des particules à base d'au moins un polymère hydrosoluble ioniquement chargé, d'au moins un tensioactif de charge ionique opposée à celle dudit polymère, d'au moins un copolymère à blocs hydrosoluble possédant au moins un bloc A hydrophile non ionique et au moins un bloc B ionique ou hydrophobe non ionique, les particules comprenant éventuellement au moins une matière active ; la structure interne des particules formant une mésophase ; les particules se trouvant en suspension dans une phase aqueuse.

Un autre objet de l'invention est constitué par un procédé de préparation des particules, dans lequel on met en oeuvre les étapes suivantes :
- On prépare un premier mélange aqueux comprenant le polymère hydrosoluble ioniquement chargé et le cas échéant le copolymère à blocs hydrosoluble si celui-ci est de même charge ;
- On prépare un deuxième mélange aqueux comprenant le tensioactif et le cas échéant, le copolymère à blocs hydrosoluble si celui-ci est de même charge que le tensioactif ou s'il ne possède pas de charge ionique ;
- On ajoute le deuxième mélange au premier.

L'invention concerne de plus l'utilisation desdites particules, comme agent modificateur d'état de surface, comme agent vectorisant de matières actives, dans des formulations destinées au traitement de la peau et/ou du cheveu, destinées au traitement des textiles, destinées au traitement et/ou à la déformation de métaux, ou encore applicables dans le domaine phytosanitaire.

L'invention a encore pour objet des formulations destinées à des applications dans les domaines mentionnés ci-dessus et comprenant les particules précitées.

Enfin, un dernier objet de l'invention est constitué par l'utilisation de copolymères à blocs hydrosolubles possédant au moins un bloc A hydrophile non ionique et au moins un bloc B ionique ou hydrophobe non ionique comme agent stabilisant de particules dont la structure interne forme une mésophase et comprenant au moins un polymère hydrosoluble ioniquement chargé, au moins un tensioactif de charge ionique opposée à celle du polymère ; la structure interne des particules résultantes, qui se trouvent en suspension dans une phase aqueuse et comprennent le polymère ioniquement chargé, le tensioactif et le copolymère à blocs, formant la même mésophase.

Les particules selon l'invention peuvent être obtenues en mettant en oeuvre un procédé simple et leur concentration en suspension est plus élevée que celle atteinte par les procédés classiques.

En outre, les particules selon l'invention présentent l'avantage d'être stables lorsqu'elles sont dispersées en milieu aqueux, et, dans des conditions appropriées, en milieu aqueux comprenant des tensioactifs.

On a par ailleurs constaté que les particules selon l'invention pouvaient comprendre une ou plusieurs matières actives. Ainsi, les particules selon l'invention peuvent être employées comme des moyens pour encapsuler et vectoriser des matières actives.

Elles peuvent de même être utilisées comme agents modificateurs de surface.

Mais d'autres avantages et caractéristiques de la présente invention apparaîtront plus clairement à la lecture de la description et des exemples qui vont suivre.

Les particules selon la présente invention, selon une première caractéristique, sont à base d'au moins un polymère hydrosoluble ioniquement chargé, d'au moins un tensioactif de charge ionique opposée à celle dudit polymère, d'au moins un copolymère à blocs hydrosoluble possédant au moins un bloc A hydrophile non ionique et au moins un bloc B ionique ou hydrophobe non ionique et éventuellement d'au moins une matière active.

Tout d'abord, le polymère hydrosoluble ioniquement chargé possède des charges positives ou des charges négatives.

Par hydrosoluble, on désigne des polymères qui, en solution dans l'eau avec une concentration comprise entre 0,01 et 1 %, à une température d'environ 20°C, ne donnent pas, dans tout ou partie de la gamme de concentration indiquée, de séparation macroscopique de phases.

De plus, le polymère ioniquement chargé est de manière avantageuse, un polymère linéaire.

Il est à noter que ledit polymère peut en outre être un homopolymère ou un copolymère statistique.

Au cas où le polymère est un copolymère, ce dernier est obtenu à partir de monomères portant le même type de charge ionique.

Conformément à un mode de réalisation particulier de l'invention, les monomères à partir desquels le polymère chargé est obtenu, sont choisis de telle façon qu'au moins 70 % en mole de ces monomères sont ioniquement chargés dans les conditions de pH de la composition et de la suspension.

A titre de monomères anioniques convenables, on peut citer entre autres les monomères comprenant au moins une fonction carboxylique, sulfonique, sulfurique, phosphonique, phosphorique, sulfosuccinique, ou les sels correspondants.

Plus particulièrement, les monomères peuvent être choisis parmi :
- les acides mono- ou poly- carboxyliques linéaires, ramifiés, cycliques ou aromatiques, comprenant au moins une insaturation éthylénique, de préférence 2 à 10 atomes de carbone et éventuellement au moins un groupement hydroxyle, les dérivés N-substitués de tels acides ; les monoesters d'acides polycarboxyliques, comprenant au moins une insaturation éthylénique, de préférence 2 à 10 atomes de carbone et éventuellement au moins un groupement hydroxyle, ;
- les acides vinyl-carboxyliques linéaires, ramifiés, cycliques ou aromatiques, comprenant 2 à 10 atomes de carbone ;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

Il est rappelé que le terme macromonomère désigne une macromolécule portant une ou plusieurs fonctions susceptibles d'être polymérisées par la méthode appropriée (par exemple, par polycondensation, par polyaddition, par voie radicalaire).

A titre d'exemples de monomères anioniques, on peut citer sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges, ainsi que les macromonomères dérivant de tels monomères.

On ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

En tant que monomères hydrophiles cationiques à partir desquels les polymères ioniquement chargés peuvent être obtenus, on peut citer notamment :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, aromatique ou non, la vinylamine, l'éthylène imine ;
- les sels d'ammonium de diallyldialkyl ;
seuls ou en mélanges, ou les sels correspondants, ainsi que les macromonomères dérivant de tels monomères.

Il est rappelé que le terme macromonomère désigne une macromolécule portant une ou plusieurs fonctions polymérisables par la méthode choisie de polymérisation.

A titre d'exemples plus précis de monomères cationiques à partir desquels le polymère utilisé selon l'invention peut être synthétisé, on peut citer notamment :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide, le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido ;
- l'éthylène imine, la vinylamine, la pyridine, la 2-vinylpyridine, la 4-vinylpyridine, la pyrrolidine, la vinyl-1 pyrrolidine-2, l'imidazoline, la vinyl-1 imidazoline-3, la vinylamine, l'éthylène imine ;
- le chlorure de triméthyl ammonium de vinylbenzyle ;
- le chlorure d'ammonium de diallyldiméthyl ;
seuls ou en mélanges, ou leurs sels correspondants, ainsi que les macromonomères dérivant de tels monomères.

Lorsque les monomères se trouvent sous forme de sels, et plus particulièrement avec des fonctions amines quatemisées, de type ammonium NR₄⁺ avec R, identiques ou non, représentant un atome d'hydrogène, un radical alkyle ou hydroxyalkyle comprenant 1 à 10 atomes de carbone, de préférence 1 à 4, éventuellement porteur d'un radical hydroxyle ; le contre-ion peut être choisi parmi les halogénures comme par exemple le chlore, les sulfates, les hydrosulfates, les alkylsulfates (par exemple comprenant 1 à 6 atomes de carbone), les phosphates, les citrates, les formates, les acétates.

Le polymère hydrosoluble ioniquement chargé peut éventuellement comprendre des monomères qui ne portent pas de charge ionique dans les conditions de pH de la composition et de la suspension, bien que cette variante ne soit pas préférée.

S'ils sont présents, ces monomères sont plus particulièrement choisis parmi les monomères hydrophiles non ioniques ou hydrophobes non ioniques.

En un tel cas de figure, ces derniers sont présents en quantité telle que le polymère ioniquement chargé soit hydrosoluble au sens indiqué précédemment.

Plus particulièrement, s'ils sont présents, le pourcentage molaire de ce type de monomères dans le polymère est inférieur ou égal à 30 %, de préférence inférieur ou égal à 10 %.

Une liste de monomères hydrophobes et hydrophiles non ioniques sera donnée ultérieurement.

Le polymère ioniquement chargé peut être obtenu en mettant en oeuvre tout type de polymérisation classique, comme les polymérisations par voie anionique, par voie cationique, par voie radicalaire dite vivante ou contrôlée. II est de même possible de mettre en oeuvre, selon les monomères employés, une polymérisation par transfert de groupe (dite "group transfert") ou bien une polymérisation par ouverture de cycle, par polyaddition, par polycondensation, ou encore par une polymérisation mettant en jeu une transestérification de groupements terminaux.

La masse molaire des polymères ioniquement chargés est de préférence comprise entre 600 et 6.106 g/mol, plus particulièrement comprise entre 2000 et 100000 g/mol, de préférence entre 2000 et 10000 g/mol. Il est indiqué que les masses molaires en poids sont des masses absolues et sont déterminées par chromatographie par exclusion stérique couplée à la méthode MALLS (Multi-Angle Laser Light Scattering).

En ce qui concerne le tensioactif entant dans la composition des particules selon l'invention, celui-ci est choisi parmi les espèces de charge ionique opposée à celle du polymère qui vient d'être décrit.

Ainsi, lorsque le polymère est anionique, le tensioactif est cationique et inversement, dans les conditions de pH de la composition et de la suspension.

Parmi les tensioactifs cationiques, on peut citer notamment les mono- ou polyamines primaires, secondaires ou tertiaires, ou possédant un ou plusieurs groupement ammonium quaternaire, comprenant plus particulièrement 6 à 40 atomes de carbone, aliphatiques linéaires ou ramifiées, aromatiques, ainsi que celles comprenant éventuellement un ou plusieurs groupement(s) alcoxylé(s) (éthoxylés et/ou propoxylés).

Plus particulièrement, on peut citer l'hexylamine, l'octylamine, la dodécylamine, la stéarylamine, l'hexadécylamine, l'oléylamine, le diaminohexane, le diaminoheptane, le diaminododécane, la benzoctamine, les halogénures d'alkyldialkylammonium ou d'alkyltrialkylammonium ou d'alkylbenzyldialkylammonium, comme le chlorure, le bromure de dodécyltriméthyl-ammonium, le chlorure, le bromure d'hexadécyl-triméthylammonium, le chlorure, le bromure de benzalkonium ;
- les sels de pipéridinium ;
- les imidazoles ;
- les amines hétérocycliques ;
seuls ou en mélange.

En ce qui concerne les tensioactifs anioniques, conviennent par exemple, seuls ou combinés :
- les alkylesters sulfonates, par exemple de formule R-CH(SO₃M)-COOR', où R représente un radical alkyle en C₈-C₃₀, de préférence en C₁₄-C₂₀, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆ ; les alkylbenzènesulfonates, plus particulièrement en C₁₄-C₂₀, les alkylsulfonates primaires ou secondaires, notamment en C₁₄-C₂₂, les alkylglycérol sulfonates, les acides polycarboxyliques sulfonés, comme par exemple ceux décrits dans GB 1082179, les sulfonates de paraffine ;
- les alkylsulfates par exemple de formule ROSO₃M, où R représente un radical alkyle ou hydroxyalkyle en C₁₀-C₂₄, de préférence en C₁₄-C₂₀ ; M représentant un atome d'hydrogène ou un cation de même définition que ci-dessus, ainsi que leurs dérivés polyalcoxylés (éthoxylés, propoxylés, ou leurs combinaisons), comme par exemple le dodécylsulfate de sodium ;
- les alkyléthersulfates par exemple de formule RO(CH₂CH₂O)ₙSO₃M où R représente un radical alkyle ou hydroxyalkyle en C₁₀-C₂₄, de préférence en C₁₄-C₂₀ ; M représentant un atome d'hydrogène ou un cation de même définition que ci-dessus, n variant généralement de 1 à 4, ainsi que leurs dérivés polyalcoxylés (éthoxylés, propoxylés, ou leurs combinaisons), comme par exemple le laurylethersulfate avec n = 2.
- les alkylamides sulfates, par exemple de formule RCONHR'OSO₃M où R représente un radical alkyle en C₂-C₂₂, de préférence en C₁₄-C₂₀, R' un radical alkyle en C₂-C₃, M représentant un atome d'hydrogène ou un cation de même définition que ci-dessus, ainsi que leurs dérivés polyalcoxylés (éthoxylés, propoxylés, ou leurs combinaisons) ;
- les sels d'acides gras saturés ou insaturés, par exemple comme ceux en C₈-C₂₄, de préférence en C₁₄-C₂₀, les N-acyl N-alkyltaurates, les alkyliséthionates, les alkylsuccinamates et alkylsulfosuccinates, les monoesters ou diesters de sulfosuccinates, les N-acyl sarcosinates, les polyéthoxycarboxylates ; et
- les phosphates esters d'alkyle et/ou d'alkyléther et/ou d'alkylaryléther.

Les tensioactifs ioniques peuvent éventuellement être associés à un ou plusieurs tensioactifs non ioniques, bien que cette variante ne soit pas préférée.

Dans un tel cas, la teneur en tensioactif non ionique est inférieure ou égale à 30 % en poids du tensioactif ionique, de préférence inférieure ou égale à 10 % en poids du tensioactif ionique.

Parmi les possibles tensioactifs non ioniques, on peut citer entre autres les alcools, les acides, de préférence en C₆-C₂₄, les mono-, di- et triglycérides, les esters de sorbitan, les amines grasses, les di et tri (phényl-1 éthyl) phénols, seuls ou en combinaison, ainsi que leurs dérivés alcoxylés (éthoxylés, ou éthoxylés/porpoxylés).

Il est de plus précisé que le choix du tensioactif ionique est fait en fonction de l'apparition d'une mésophase lorsqu'il est associé au polymère ioniquement chargé, dans les mêmes conditions de concentrations que celle des particules selon l'invention (c'est-à-dire avec des concentrations respectives en polymère ioniquement chargé et tensioactif identiques à celles utilisées pour préparer les particules selon l'invention, le copolymère à blocs étant exclu).

Par mésophase, on désigne les phases organisées de complexes polymère chargé / tensioactif de charge opposé, comme par exemple des phases lamellaires, hexagonale, cubique, bi-continue (éponge). De préférence, le complexe polymère chargé / tensioactif de charge opposée est de type lamellaire.

Précisons que lorsque l'on utilise dans le cadre de l'invention, les termes "même mésophase", cela signifie effectivement que la même mésophase est conservée, mais aussi qu'elle peut se trouver sous la forme d'une mésophase de même type. A titre illustratif, une mésophase lamellaire et une mésophase sphérulitique (onions) seront considérées comme étant, au sens de l'invention, une même mésophase.

Il est aisé pour l'homme du métier d'établir quelle association de polymère chargé et de tensioactif de charge opposée peut donner un complexe présentant une structure interne organisée. En effet, il lui suffit d'établir le diagramme de phase de l'ensemble polymère, tensioactif et eau, de constater si une phase organisée apparaît dans le diagramme ternaire, et si oui, de déterminer la composition qui permet de l'obtenir. Ladite phase organisée est généralement en équilibre avec une phase diluée non organisée, riche en eau, et se présente sous la forme d'une phase macroscopique qui n'est pas utilisable.

L'une des caractéristiques essentielles de la présente invention réside dans la présence d'au moins un copolymère à blocs hydrosoluble possédant au moins un bloc A hydrophile non ionique et au moins un bloc B ionique ou hydrophobe non ionique.

En effet, et cela représente un autre objet de la présente invention, on a remarqué d'une manière totalement inattendue, que l'utilisation de copolymères à blocs de ce type permettait de disperser la mésophase mentionnée auparavant, sous la forme d'une dispersion stable de particules de taille nanométrique ou micrométrique.

En d'autres termes, la présence du copolymère à blocs permet d'obtenir une suspension stable de particules, alors que l'ensemble polymère chargé / tensioactif de charge opposée, utilisé seul, aurait conduit à une séparation macroscopique de phases.

En outre, on a observé que la structure interne des particules comprenant le polymère chargé, le tensioactif et le copolymère à blocs, formait la même mésophase (au sens mentionné plus haut) que celle obtenue sans le copolymère à blocs.

Ainsi, le copolymère à blocs apporte une autre caractéristique totalement inattendue, qui est celle de conserver la phase organisée qui existait initialement entre le polymère chargé et le tensioactif de charge opposée. Ceci constitue un avantage supplémentaire de la présente invention car la phase organisée elle-même peut apporter des caractéristiques intéressantes en termes d'applications futures des particules (lubrification, encapsulation).

Enfin la présence de ce copolymère à blocs permet d'obtenir des particules présentant une taille moyenne relativement faible. Avantageusement, les particules selon l'invention présentent une taille moyenne comprise entre 5 nanomètres et 50 µm. Les tailles moyennes sont mesurées plus précisément au moyen d'un granulomètre Horiba, et correspondent au diamètre médian en volume (d50) qui représente le diamètre de la particule égal à 50 % de la distribution cumulative.

Comme cela a été indiqué plus haut, le copolymère à blocs est hydrosoluble. Rappelons qu'un polymère est dit hydrosoluble lorsqu'il ne donne pas de séparation macroscopique de phases, dans tout ou partie d'une gamme de concentration dans l'eau comprise entre 0,01 et 1 % en poids, à environ 20°C.

Par ailleurs, le copolymère à blocs peut avoir une structure linéaire (multiblocs), ramifiée (peigne ou greffée) ou encore étoile.

Les copolymères à blocs linéaires ont plus particulièrement une structure comprenant au moins deux blocs (diblocs).

Les copolymères à blocs de structure ramifiée (peigne ou greffé) présentent, de manière préférentielle, un squelette ioniquement chargé ou hydrophobe sur lequel sont greffés des segments hydrophiles neutres.

En ce qui concerne les copolymères à blocs de structure étoile, plusieurs possibilités sont envisageables. Selon un mode de réalisation particulier, si l'on considère chaque branche de l'étoile, celle-ci peut comprendre soit un copolymère à blocs, de préférence un dibloc, dont l'un est un bloc hydrophile neutre (A), l'autre un bloc ionique ou hydrophobe (B) ; soit un bloc (A) ou (B).

Les copolymères à blocs qui viennent d'être détaillés sont des composés bien connus de l'homme de l'art.

Ainsi, les copolymères peuvent être préparés en mettant en oeuvre des polymérisations par voie anionique, par voie cationique, par voie radicalaire dite vivante ou contrôlée. Il est de même possible de mettre en oeuvre, selon les monomères employés, une polymérisation par transfert de groupe ou bien une polymérisation par ouverture de cycle (notamment anionique ou cationique), ou encore par une polymérisation mettant en jeu une transestérification de groupements terminaux.

De préférence, les polymères sont obtenus en mettant en oeuvre au moins une étape de polymérisation radicalaire vivante.

A titre d'exemple de procédés de polymérisation dite vivante ou contrôlée, on peut notamment se référer à la polymérisation radicalaire contrôlée par les xanthates selon l'enseignement de la demande WO 98/58974, la polymérisation radicalaire contrôlée par les dithioesters selon l'enseignement de la demande WO 97/01478, la polymérisation à l'aide de précurseurs nitroxydes selon l'enseignement de la demande WO 99/03894, la polymérisation radicalaire contrôlée par les dithiocarbamates selon l'enseignement de la demande WO 99/31144, la polymérisation radicalaire par transfert d'atome (ATRP) selon l'enseignement de la demande WO 96/30421.

Les polymères greffés ou peignes peuvent être obtenus par des méthodes dites de greffage direct et copolymérisation.

Le greffage direct consiste à polymériser le(s) monomère(s) choisi(s) par voie radicalaire, en présence du polymère sélectionné pour former le squelette du produit final. Si le couple monomère/squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.

Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".

Dans le cas de polymères de type étoile, les synthèses peuvent être essentiellement classées en deux groupes. Le premier correspond à la formation des bras des polymères à partir d'un composé plurifonctionnel constituant le centre (technique "core-first") (Kennedy, J.P. and coll. Macromolecules, 29, 8631 (1996), Deffieux, A. and coll. lbid, 25, 6744, (1992), Gnanou, Y. and coll. Ibid, 31, 6748 (1998)) et le second correspond à une méthode où les molécules de polymères qui vont constituer les bras sont d'abord synthétisées et ensuite liées ensemble sur un coeur pour former un polymère en forme d'étoile (technique "arm-first"). Parmi les méthodes utilisables pour lier les bras, on peut notamment citer la méthode comprenant la réaction de ces bras avec un composé présentant une pluralité de groupes fonctionnels capables de réagir avec des groupements fonctionnels antagonistes terminaux desdits bras (Fetters, L.J. and coll. Macromolecules, 19, 215 (1986), Hadjichristidis, N. and coll. Macromolecules, 26, 2479 (1993), Roovers, J. and coll. Macromolecules, 26, 4324 (1993)). Citons également la méthode comprenant l'ajout d'un composé présentant une pluralité de groupes polymérisables, suivi de la polymérisation desdits bras (Rempp, P. and coll., Polym. Sci. Part C, 22, 145 (1968), Fetters, L. J. and coll. Macromolecules, 8, 90 (1975), Higashimura and coll. Ibid, 24, 2309 (1991)).

Pour obtenir les chaînes polymères constituant ultérieurement les bras des étoiles, on a généralement recours à des méthodes permettant de contrôler la réaction de polymérisation. Ainsi, les polymérisations anionique et cationique vivante sont les méthodes les plus utilisées actuellement.

De préférence, le copolymère à blocs mis en oeuvre selon l'invention, sont des copolymères linéaires comprenant deux blocs.

En ce qui concerne le bloc A, celui-ci est obtenu à partir de monomères hydrophiles non ioniques, plus particulièrement choisis parmi l'oxyde d'éthylène ; les amides des acides mono- ou poly- carboxyliques, linéaires, ramifiés, cycliques ou aromatiques, comprenant au moins une insaturation éthylénique ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate^{®} de vinyle, le propionate de vinyle ; les monomères du type des sucres comme les osides, les polyholosides fortement dépolymérisés.

Par fortement dépolymérisés, on entend des composés dont la masse moléculaire en poids est plus particulièrement inférieure à 20000 g/mole (déterminée par chromatographie par exclusion stérique couplée à la méthode MALLS (Multi-Angle Laser Light Scattering).

Les osides sont des composés qui résultent de la condensation, avec élimination d'eau, de molécules d'oses entre elles ou encore de molécules d'oses avec des molécules non glucidiques. Parmi les osides on préfère les holosides qui sont formés par la réunion de motifs exclusivement glucidiques et plus particulièrement les oligoholosides (ou oligosaccharides) qui ne comportent qu'un nombre restreint de ces motifs, c'est-à-dire un nombre en général inférieur ou égal à 10. A titre d'exemples d'oligoholosides on peut mentionner le saccharose, le lactose, la cellobiose, le maltose.

Les polyholosides (ou polysaccharides) fortement dépolymérisés convenables sont décrits par exemple dans l'ouvrage de P. ARNAUD intitulé "cours de chimie organique", GAUTHIER-VILLARS éditeurs, 1987. A titre d'exemple non limitatif de polyholosides fortement dépolymérisés, on peut citer le dextran, l'amidon.

Il est à noter que les monomères précités peuvent aussi être mis en oeuvre sous la forme de macromonomères.

Pour ce qui a trait au bloc B, l'une des possibilités de la présente invention est que ce bloc est obtenu à partir de monomères hydrophobes non ioniques.

Parmi les monomères convenables, figurent entre autres :
- les esters des acides mono- ou poly- carboxyliques, linéaires, ramifiés, cycliques ou aromatiques, comprenant au moins une insaturation éthylénique ;
- les nitriles αβ-éthyléniquement insaturés, les éthers vinyliques, les esters vinyliques, les monomères vinylaromatiques, les halogénures de vinyle ou de vinylidène ;
- les monomères hydrocarbonés, linéaires ou ramifiés, aromatiques ou non, comprenant au moins une insaturation éthylénique ; seuls ou en mélanges, ainsi que les macromonomères dérivant de tels monomères

A titre d'exemples particuliers de monomères utilisables dans d'entrer dans la préparation du ou des blocs hydrophobes des copolymères à blocs, on peut citer :
- les esters d'acide (méth)acrylique avec un alcool comprenant 1 à 12 atomes de carbone comme le (méth)acrylate de méthyle, le (méth)acrylate d'éthyle, le (méth)acrylate de propyle, le (méth)acrylate de n-butyle, le (méth)acrylate de t-butyle, le (méth)acrylate d'isobutyle, l'acrylate de 2-éthylhexyl ;
- l'acétate de vinyle, le Versatate^{®} de vinyle, le propionate de vinyle, le chlorure de vinyle, le chlorure de vinylidène, le méthyl vinyléther, l'éthyl vinyléther ;
- les nitriles vinyliques incluent plus particulièrement ceux ayant de 3 à 12 atomes de carbone, comme en particulier l'acrylonitrile et le méthacrylonitrile ;
- le styrène, l'α-méthylstyrène, le vinyltoluène, le butadiène, le chloroprène ; seuls ou en mélanges, ainsi que les macromonomères dérivant de tels monomères.

Les monomères préférés sont les esters de l'acide acryliques avec les alcools linéaires ou ramifiés en C₁-C₄ tels que le (méth)acrylate de méthyle, d'éthyle, de propyle et de butyle, les esters vinyliques comme l'acétate de vinyle, le styrène, l'α-méthylstyrène.

Selon une autre possibilité de l'invention, le bloc B du copolymère à blocs est obtenu à partir de monomères ioniques.

Une première catégorie de monomères ioniques est constituée par des monomères cationiques.

A titre d'exemples de monomères de ce type, on peut citer notamment :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges, les sels correspondants, ainsi que les macromonomères dérivant de tels monomères.

Une deuxième catégorie de monomères ioniques est constituée par des monomères anioniques.

Plus particulièrement, on pourra utiliser des monomères anioniques comprenant au moins une fonction carboxylique, sulfonique, sulfurique, phosphonique, phosphorique, sulfosuccinique, les sels correspondants, ainsi que les macromonomères dérivant de tels monomères.

De manière avantageuse, les monomères anioniques sont choisis parmi :
- les acides mono- ou poly- carboxyliques linéaires, ramifiés, cycliques ou aromatiques, comprenant au moins une insaturation éthylénique, de préférence 2 à 10 atomes de carbone et éventuellement au moins un groupement hydroxyle, les dérivés N-substitués de tels acides ; les monoesters d'acides polycarboxyliques, comprenant au moins une insaturation éthylénique, de préférence 2 à 10 atomes de carbone et éventuellement au moins un groupement hydroxyle, ;
- les acides vinyl-carboxyliques linéaires, ramifiés, cycliques ou aromatiques, comprenant 2 à 10 atomes de carbone ;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

Les monomères anioniques et cationiques ont fait l'objet d'une description plus exhaustive dans la partie relative au polymère chargé ioniquement, et l'on pourra s'y référer.

La composition des blocs A et B du copolymère à blocs peut varier dans un large domaine, dès l'instant que ledit copolymère est hydrosoluble au sens de l'invention.

Il est de plus à noter que selon un mode de réalisation particulier de l'invention, le copolymère à blocs présente une masse molaire en poids comprise entre 600 et 106 g/mol, de préférence comprise entre 2000 et 100000 g/mol. Les masses molaires en poids sont des masses absolues déterminées par chromatographie par exclusion stérique couplée à la méthode MALLS.

Par ailleurs, la masse et le nombre de blocs hydrophiles neutre sont adaptés de manière à ce que le copolymère à blocs soit hydrosoluble et assure de plus, la dispersion des particules dans la phase aqueuse. Ainsi, de manière avantageuse, le rapport des masses molaires des blocs A et des blocs B (blocs A / blocs B) est de préférence supérieur ou égal à 2. Les masses molaires sont des masses molaires en poids, théoriques, c'est-à-dire attendues à l'issue de la polymérisation ; elles sont évaluées en fonction de la quantité des monomères mis en oeuvre dans la réaction de polymérisation.

Selon une caractéristique plus particulière de la présente invention, la dispersion aqueuse de particules comprend une quantité de polymère ioniquement chargé comprise entre 0,01 et 30 % en poids par rapport à la phase aqueuse, plus particulièrement entre 0,05 et 10 % en poids ; de préférence entre 0,05 et 2 % en poids.

La quantité de tensioactif est plus particulièrement exprimée en fonction du rapport Zs qui correspond à la concentration molaire de charge du tensioactif divisée par la concentration molaire de charge du polymère ioniquement chargé.

Selon ce critère, la quantité de tensioactif est de préférence telle que le rapport Zs est compris entre 0,01 et 100, plus particulièrement entre 0,1 et 10, de préférence entre 1 et 5.

Quant à la quantité de copolymère à blocs entrant dans la dispersion aqueuse de particules selon l'invention, elle sera exprimée différemment selon que le copolymère comprend ou non un ou plusieurs blocs ioniquement chargés, et selon que la charge ionique est cationique ou anionique.

Dans le cas où le copolymère à blocs est de même charge que le polymère ioniquement chargé, alors la quantité de copolymère à blocs est déterminée avantageusement en fonction du rapport Zb, correspondant à la concentration molaire de charge du tensioactif divisée par la concentration molaire de charge du polymère ioniquement chargé et du copolymère à blocs. Selon ce critère, la quantité de copolymère à blocs est plus particulièrement telle que le rapport Zb est compris entre 0,5 et 1,5 ; avantageusement entre 0,8 et 1,2.

Dans le cas où le copolymère à blocs est de charge opposée à celle du polymère ioniquement chargé, la quantité de copolymère à blocs est déterminée avantageusement en fonction du rapport Z'b, correspondant à la concentration molaire de charge du tensioactif et du copolymère à blocs, divisée par la concentration molaire en charge du polymère ioniquement chargé, est compris entre 0,5 et 1,5 ; plus particulièrement entre 0,8 et 1,2.

Enfin, dans le cas où le copolymère à blocs ne possède pas de charge ionique, la teneur en ce copolymère est plus particulièrement comprise entre 10 et 100% en poids par rapport au poids de polymère ioniquement chargé et de tensioactif. Dans le cadre de cette possibilité, le rapport Zs, correspondant à la concentration molaire de charge du tensioactif divisée par la concentration molaire en charge du polymère ioniquement chargé, est plus compris entre 0,5 et 1,5 ; plus particulièrement entre 0,8 et 1,2.

Comme cela a été indiqué auparavant, les particules selon l'invention peuvent comprendre au moins une matière active.

Toutes les substances apportant un effet dans l'application ultérieure des particules peuvent être mises en oeuvre, dès l'instant qu'elles sont peu ou non miscibles dans l'eau. Par peu ou non miscibles, on entend des substances dont la solubilité dans l'eau ne dépasse pas 10 % en poids, à environ 20°C, des particules selon l'invention.

Ces matières actives peuvent se présenter indifféremment sous une forme liquide, solubilisée dans un solvant organique peu ou non miscible à l'eau au sens indiqué précédemment, ou encore sous une forme solide, à environ 20°C, des particules selon l'invention.

En outre, les matières actives présentent de préférence une température de fusion inférieure ou égale à 100°C, plus particulièrement inférieure ou égale à 80°C.

En tant que composé convenable, on peut citer notamment les huiles organiques, d'origine animale ou végétale, ou minérales, ainsi que les cires provenant des mêmes origines, ou leurs mélanges.

Comme huiles organiques d'origine animale, on peut citer entre autres, l'huile de cachalot, l'huile de baleine, l'huile de phoque, l'huile de sardine, l'huile de hareng, l'huile de squale, l'huile de foie de morue ; les graisses de porc, de mouton (suifs).

En tant que cires d'origine animale, on peut citer la cire d'abeille.

A titres d'exemples d'huiles organiques d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin, le beurre de cacao, le beurre de karité.

En tant que cires d'origine végétale, on peut citer la cire de carnauba.

En ce qui concerne les huiles minérales, on peut citer entre autres les coupes pétrolières, les huiles naphténiques, paraffiniques (vaseline). Les cires paraffiniques peuvent de même convenir à la préparation de l'émulsion.

Les produits issus de l'alcoolyse des huiles précitées peuvent aussi être utilisés.

On ne sortirait du cadre de la présente invention en mettant en oeuvre, en tant que matière active, au moins un acide gras, saturé ou non; au moins un alcool gras, saturé ou non, au moins un ester d'acide gras, ou leurs mélanges.

Plus particulièrement, lesdits acides comprennent 8 à 40 atomes de carbone, plus particulièrement 10 à 40 atomes de carbone, de préférence 18 à 40 atomes de carbone, et peuvent comprendre une ou plusieurs insaturations éthyléniques, conjuguées ou non, et éventuellement un ou plusieurs groupements hydroxyles. Quant aux alcools, ils peuvent comprendre un ou plusieurs groupements hydroxyles.

Comme exemples d'acides gras saturés, on peut citer les acides palmitique, stéarique, isostéarique, béhénique.

Comme exemples d'acides gras insaturés, on peut citer les acides myristoléique, palmitoléique, oléique, érucique, linoléique, linolénique, arachidonique, ricinoléique, ainsi que leurs mélanges.

Concernant les esters d'acides gras, ceux-ci peuvent avantageusement être obtenus à partir d'acides gras précités et d'alcool comprenant notamment 1 à 6 atomes de carbone. De préférence, il s'agit d'esters méthylique, éthylique, propylique, isopropylique.

Quant aux alcools, ceux-ci comprennent plus particulièrement 4 à 40 atomes de carbone, de préférence 10 à 40 atomes de carbone, éventuellement une ou plusieurs insaturations éthyléniques, conjuguées ou non, et éventuellement plusieurs groupements hydroxyles. Les polymères comprenant plusieurs groupement hydroxyles peuvent de même convenir, comme par exemple les polypropylèneglycols.

Comme exemple d'alcools, on peut citer par exemple ceux correspondants aux acides précités.

A titre d'exemple de matières actives dans le domaine de l'alimentaire, on peut citer les mono-, di- et triglycérides, les huiles essentielles, les arômes, les colorants.

A titre d'exemple de matières actives utilisables dans le domaine de la cosmétique on peut citer les huiles silicones appartenant par exemple à la famille des diméthicones ; la co-enzyme Q10 ; les vitamines lipophiles, comme la vitamine A et ses dérivés notamment ses esters comme l'acétate, le palmitate, le propionate, la vitamine B2, l'acide pantothénique, la vitamine D et la vitamine E ; les mono-, di- et triglycérides ; les bactéricides ; les agents absorbeurs d'UV, comme les dérivés aminobenzoate de type PABA et PARA, les salicylates, les cinnamates, les anthranilates, les dibenzoylméthanes, les dérivés du camphre et leurs mélanges.

Les agents anti-vieillissement peuvent de même être utilisés. A titre d'exemples de tels agents on peut citer notamment citer les rétinoïdes, les vitamines liposolubles, les dérivés de la vitamine C comme les esters notamment l'acétate, le propionate, le palmitate ; les céramides, les pseudo-céramides, les phospholipides, les acides gras, les alcools gras, le cholestérol, les stérols et leurs mélanges. Comme acides gras et alcools préférés, on peut plus particulièrement citer ceux possédant des chaînes alkyles, linéaires ou ramifiées contenant de 12 à 20 atomes de carbone. Il peut notamment s'agir d'acide linoléique.

On peut de même mettre en oeuvre des agents anti-cellulite, tels que notamment l'isobutylméthylxanthine et la théophyline ; ainsi que des agents anti-acné, comme par exemple le résorcinol, l'acétate de résorcinol, le peroxyde benzoyle et de nombreux composés naturels.

Les arômes, huiles essentielles, parfums peuvent aussi être utilisés en tant que matière active hydrophobe. On pourra se référer aux listes de composés indiquées auparavant.

Les agents anti-microbiens peuvent être choisis parmi le thymol, le menthol, le triclosan, le 4-hexylrésorcinol, le phénol, l'eucalyptol, l'acide benzoïque, le peroxyde benzoïque, le parabène de butyle, et leurs mélanges.

A titre d'exemple de matières actives convenables pour la réalisation de l'invention, dans le domaine des peintures, on peut citer les résines alkydes, les résines époxy, les isocyanates masqués ou non.

Dans le domaine du papier, on peut citer à titre d'exemple les résines de collage et d'hydrofugation telles que le dimère d'alkylcétène (AKD) ou l'anhydride alcényle succinique (ASA).

Dans le domaine de l'agrochimie, les matières actives phytosanitaires peuvent être choisies parmi la famille des α-cyano-phénoxybenzyl carboxylates ou des α-cyano-halogénophénoxy-carboxylates, la famille des N-méthylcarbonates comprenant des substituants aromatiques, les matières actives telles que Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, Cyhalothrin.

Dans le domaine de la détergence, on peut mentionner en tant que matière active possible les antimousses silicones.

Il est de même possible d'utiliser des matières actives telles que celles entrant dans la composition de lubrifiants pour le travail ou la déformation des matériaux. La matière active est habituellement une huile, un dérivé d'une huile ou encore un ester d'acide gras ou un sel d'acide gras, tels que ceux mentionnés auparavant.

La matière active peut aussi être choisie parmi les solvants organiques ou les mélanges de tels solvants pas ou peu miscibles dans l'eau, comme notamment ceux mis en oeuvre pour le nettoyage ou le décapage, tels que les coupes pétrolières aromatiques, les composés terpéniques comme les D- ou L- limonènes, ainsi que les solvants comme le Solvesso^{®}. Conviennent aussi comme solvants, les esters aliphatiques, comme les esters méthyliques d'un mélange d'acides acétique, succinique et glutarique (mélange d'acides sous-produit de la synthèse du Nylon), les huiles comme l'huile de vaseline, et les solvants chlorés.

Si une ou plusieurs matières actives sont présentes, elles représentent plus particulièrement une quantité comprise entre 0,01 et 100% en poids par rapport au poids de polymère ioniquement chargé, de tensioactif et de copolymère à blocs.

La dispersion de particules comprend de manière avantageuse une concentration en particules comprise entre 0,1 et 50 % en poids par rapport la phase aqueuse, de préférence entre 0,1 et 30 % en poids par rapport la phase aqueuse.

Les particules selon l'invention présentent de plus une taille moyenne comprise entre 5 nanomètres et 50 µm, de préférence entre 50 nm et 5 µm. Les tailles moyennes sont mesurées plus précisément au moyen d'un granulomètre Horiba, et correspondent au diamètre médian en volume (d50) qui représente le diamètre de la particule égal à 50 % de la distribution cumulative.

Un deuxième objet de l'invention est constitué par un procédé de préparation des particules qui viennent d'être détaillées, consistant à mettre en oeuvre les étapes suivantes :
- On prépare un premier mélange aqueux comprenant le polymère ioniquement chargé et le cas échéant le copolymère à blocs si celui-ci est de même charge ;
- On prépare un deuxième mélange aqueux comprenant le tensioactif et le cas échéant, le copolymère à blocs si celui-ci est de même charge que le tensioactif ou s'il ne possède pas de charge ionique ;
- On ajoute le deuxième mélange au premier.

Il est à noter que si une ou plusieurs matières actives sont employées, cette ou ces dernières peuvent être introduites de plusieurs manières.

Selon une première possibilité, la ou les matières actives sont introduites dans le deuxième mélange aqueux (comprenant le tensioactif).

Selon une deuxième possibilité, la ou les matières actives sont introduites une fois réalisé l'ajout du deuxième mélange au premier.

Une combinaison de ces deux possibilités est envisageable.

Selon un mode de réalisation de l'invention, la température à laquelle sont préparés les mélanges est supérieure ou égale à la température à laquelle on obtient des solutions aqueuses. Ainsi, la température est choisie de telle sorte que le tensioactif soit soluble dans le mélange aqueux.

Il est par ailleurs à noter que dans le cas où une matière active est présente dans le mélange comprenant le tensioactif, la température du mélange est de préférence telle que ladite matière active se trouve sous la forme d'un liquide, afin d'en optimiser la dispersion.

A titre d'illustration, la température est supérieure à la température ambiante, de préférence supérieure ou égale à 20°C, et de manière encore plus préférée, comprise entre 20 et 90°C.

Avantageusement, la température des deux mélanges aqueux précités, de même que celle à laquelle les deux mélanges sont mis en contact, est la même ou voisine.

Au cas où une matière active est incorporée une fois la dispersion obtenue (c'est-à-dire lorsque les deux mélanges mis en contact), la température de la matière active est de préférence telle que celle-ci se trouve sous la forme d'un liquide. Il est de plus précisé que pour améliorer l'incorporation et l'homogénéité de la répartition de la matière active, la température de la dispersion est voisine de celle de la matière active.

En outre, le pH auquel la mise en contact des deux mélanges est effectuée, correspond de préférence à une valeur à laquelle la mésophase entre le polymère chargé et le tensioactif, en présence du copolymère à blocs, apparaît.

Habituellement, le pH est ajusté si nécessaire avant la mise en contact des espèces entre elles. Cependant, il n'est pas exclu que l'ajustement du pH soit fait une fois la mise en contact réalisée.

L'ajustement du pH se fait de manière totalement classique, par ajout de base (comme les hydroxydes de métal alcalin, les carbonates et bicarbonates de métal alcalin) ou bien d'acide (comme les acides minéraux du type de l'acide chlorhydrique par exemple).

La mise en contact des deux mélanges est réalisée de manière habituelle, sous agitation.

On obtient à l'issue de l'opération de mise en contact, des particules en suspension dans une phase aqueuse.

Une fois le mélange réalisé et la matière active éventuellement incorporée, on refroidit le mélange résultant si la température à laquelle il a été réalisé est supérieure ou égale à la température ambiante. Cette opération peut être réalisée en laissant refroidir le mélange sans intervention particulière, ou bien encore en abaissant activement la température du mélange (trempe par exemple).

La présente invention a de même pour objet l'utilisation des particules comme agent modificateur d'état de surface.

Elle a aussi pour objet l'utilisation des particules comme agent vectorisant de matières actives.

En effet, leur structure particulière rend possible l'encapsulation de matières actives, ainsi que leur libération. Cette libération peut avoir lieu sous l'action de divers déclencheurs, comme entre autres, des variations de pH, le cisaillement

Les particules selon l'invention peuvent être employées en tant qu'élément constitutif de formulations destinées au traitement de la peau et/ou du cheveu, au traitement de textiles, au traitement et/ou à la déformation de métaux, au domaine phytosanitaire.

Elles présentent en effet, dans certaines conditions, l'avantage de rester sous la forme de suspensions stables alors qu'elles sont introduites dans des formulations présentant des teneurs non négligeables en tensioactifs appropriés.

L'invention a de plus pour objet des formulations comprenant les particules selon l'invention, destinées au traitement de la peau et/ou du cheveu, au traitement et/ou à la déformation de métaux, au traitement des plantes (phytosanitaire), à l'industrie papetière, etc.

Des exemples concrets mais non limitatifs de l'invention vont maintenant être présentés.

### EXEMPLE

### 1/ Essai comparatif

La préparation du complexe de polyéthylène-imine et d'acide dodécanoïque est effectuée comme suit :
Le coefficient Zs (charge ionique acide / charge ionique polymère) est de 0,5.
200,3 mg d'acide dodécanoïque sont dissous dans 10 ml d'éthanol.
On ajoute alors lentement, à température ambiante, 88,0 mg de de polyéthylène-imine (Aldrich ; ref. 40,872-7) sous la forme d'une solution aqueuse à 5 % en poids dans l'eau, au mélange précité.
La solution transparente résultante est ensuite agitée pendant 30 minutes puis séchée à température ambiante, sous la forme d'un film qui comprend le complexe polyéthylène-imine / acide dodécanoïque.

L'obtention des particules est réalisée comme suit :
On prend 20 mg du complexe obtenu précédemment que l'on dissous dans 15 ml de tétrahydrofurane (THF).
On introduit ensuite 20 ml d'eau et le mélange résultant est ensuite laissé à température ambiante jusqu'à ce que l'odeur du THF ne puisse plus être détectée.
On détermine par pesée la quantité d'eau perdue au cours de l'évaporation du THF et l'on ajoute une quantité d'eau correspondante.
La concentration en solide dans la dispersion est de 0,1 % en poids.

On récupère ensuite une dispersion aqueuse comprenant des particules dispersées.

La caractérisation des particules obtenues est effectuée par une analyse par Cryo-TEM (microscopie électronique à transmission).

On montre que les particules dispersées sont sphériques, que leur taille moyenne est d'environ 90 nm, et que leur structure interne est de type onion faiblement ordonnée.

L'analyse diffraction RX aux petits angles confirme la structure de type onion.

Il n'est pas possible de reproduire une telle méthode avec des concentrations supérieures à 0,2 %.

Par ailleurs, lorsque le coefficient Zs est voisin de 1, il n'est pas possible de redisperser les particules obtenues.

### 21 Essai selon l'invention

Préparation du copolymère cationique à blocs :
(polydiméthylaminoéthyl acrylate)₁₁₀₀₀- bloc (acrylamide)₃₀₀₀₀.

### - Première étape:

### Synthèse de (poly(méthylsulfate de (2-acryloxy)éthyl) triméthylammonium)_{11K)}

Une solution aqueuse de méthylsulfate de (2-acryloxy)éthyl) triméthyl ammonium (ou TMAEAMS ; en solution à 80 % dans l'eau) est introduite dans un réacteur et la solution chauffée à 70°C.

Un mélange de S-éthylpropionyl O-éthyl xanthate (ou xanthate), d'acide 4,4-azo-bis-4-cyanovalérique (ou ACVA ; 30% molaire par rapport au xanthate) et d'isopropanol est alors introduit. Le mélange ainsi obtenu est agité pendant 12 heures à 70°C.

| Masse des réactifs introduits | | | | |
|---|---|---|---|---|
| TMAEAMS | Eau | Xanthate | ACVA | Isopropanol |
| 6,69g | 8,92g | 0,103g | 0,042g | 1,75g |

### - Seconde étape:

### Synthèse du cpolomyère polyTMAEAMS₁₁ₖ-PAM₃₀ₖ

L'ACVA (50% molaire par rapport au xanthate) dissout dans l'eau est ajouté au mélange précédent.

| Masse des réactifs introduits | |
|---|---|
| ACVA | Eau |
| 0,07g | 26,28g |

L'acrylamide, dissout dans l'eau (I), est alors ajouté de façon continue en 3 heures.

Après la première heure, l'ACVA (22% molaire par rapport au xanthate) dissout dans l'eau (II) est également ajouté.

| Masse des réactifs introduits | | | |
|---|---|---|---|
| Acrylamide | eau (I) | ACVA | eau (II) |
| 14,6g | 41,5g | 0,03g | 0,05g |

Après la deuxième heure, l'ACVA (22% molaire par rapport au xanthate) dissout dans l'eau (II) est également ajouté.

| Masse des réactifs introduits | |
|---|---|
| ACVA | Eau |
| 0,03g | 0,05g |

Après les 3 heures, le mélange est laissé sous agitation à 70°C pendant 2 heures supplémentaires.

L'extrait sec final de la solution est de 20%.

### L'obtention des particules est réalisée en mettant en oeuvre les étapes suivantes :

On prépare un premier mélange aqueux comprenant 0,1 g du copolymère obtenu précédemment et 0,1 g de polyéthylène-imine (Aldrich ; haute masse molaire ; ref. 40,872-7) dans 30 ml d'eau.

Le pH de l'ensemble est compris entre 7 et 8.

On prépare un second mélange aqueux comprenant 0,48 g d'acide dodécanoïque dans 30 ml d'eau à 45°C. Le pH de ce mélange est d'environ 7 à 8.

Le coefficient Zs est de 1,04 et le coefficient Zb est voisin de 1.

Sous agitation, on introduit lentement le deuxième mélange dans le premier.

Le rapport théorique des charges opposées est de 1.

On obtient alors une dispersion stable du type d'un latex avec une teneur en solide de 1,1 % en poids.

La caractérisation des particules obtenues est effectuée par une analyse par Cryo-TEM.

On montre que les particules sont plutôt de forme éllipsoïdale, avec une taille moyenne d'environ 310 nm.

Par ailleurs, la structure interne des particules est lamellaire mais non de type onion. On observe un empilement lamellaire non incurvé.

L'analyse diffraction RX aux petits angles confirme ceci et montre que la structure interne est ordonnée avec des distances de répétition d'environ 4 nm.

## Revendications

1. Suspension en phase aqueuse de particules à base d'au moins un polymère hydrosoluble ioniquement chargé, d'au moins un tensioactif de charge ionique opposée à celle dudit polymère, d'au moins un copolymère à blocs hydrosoluble possédant au moins un bloc A hydrophile non ionique et au moins un bloc B ionique ou hydrophobe non ionique, les particules comprenant éventuellement au moins une matière active ; la structure interne des particules formant une mésophase.

2. Suspension selon l'une des revendications précédentes, **caractérisées en ce que** le polymère ioniquement chargé est un homopolymère.

3. Suspension selon l'une des revendications précédentes, **caractérisées en ce que** le polymère ioniquement chargé est un copolymère statistique.

4. Suspension selon la revendication précédente, **caractérisées en ce que** le polymère ioniquement chargé est obtenu à partir de monomères portant le même type de charge ionique et dont au moins 80 % molaire sont ioniquement chargés dans les conditions de pH de la composition et de la suspension.

5. Suspension selon l'une des revendications précédentes, **caractérisées en ce que** le polymère ioniquement chargé est obtenu à partir de monomères hydrophiles non ioniques ou hydrophobes non ioniques ; le pourcentage molaire de ce type de monomères dans le polymère est inférieur ou égal à 30 %, de préférence inférieur ou égal à 10 %.

6. Suspension selon l'une des revendications précédentes, **caractérisées en ce que** le tensioactif est choisi parmi les tensioactifs anioniques ou cationiques, et **en ce qu'**il peut comprendre un ou plusieurs tensioactifs non ioniques à raison d'une teneur de moins de 30 % molaire par rapport au tensioactif ionique, de préférence moins de 10 % molaire.

7. Suspension selon l'une des revendications précédentes, **caractérisée en ce que** le polymère ioniquement chargé et le tensioactif forment une mésophase, dans les mêmes conditions de concentrations que celles des particules selon l'invention.

8. Suspension selon l'une des revendications précédentes, **caractérisées en ce que** les monomères à partir desquels sont formés le ou les blocs A sont choisis parmi l'oxyde d'éthylène ; les amides des acides mono- ou poly- carboxyliques, linéaires, ramifiés, cycliques ou aromatiques, comprenant au moins une insaturation éthylénique ou dérivés ; les esters hydrophiles dérivant de l'acide (méth)acrylique ; les esters vinyliques permettant d'obtenir des blocs alcool polyvinylique après hydrolyse ; les monomères du type des sucres ; seuls ou en mélanges, ainsi que les macromonomères dérivant de tels monomères.

9. Suspension selon l'une des revendications précédentes, **caractérisé en ce que** les monomères à partir desquels sont formés le ou les blocs B hydrophobes non ioniques sont choisis parmi :
- les esters des acides mono- ou poly- carboxyliques, linéaires, ramifiés, cycliques ou aromatiques, comprenant au moins une insaturation éthylénique ;
- les nitriles αβ-éthyléniquement insaturés, les éthers vinyliques, les esters vinyliques, les monomères vinylaromatiques, les halogénures de vinyle ou de vinylidène ;
- les monomères hydrocarbonés, linéaires ou ramifiés, aromatiques ou non, comprenant au moins une insaturation éthylénique ; seuls ou en mélanges, ainsi que les macromonomères dérivant de tels monomères.

10. Suspension selon l'une des revendications 1 à 8, **caractérisées en ce que** les monomères à partir desquels sont formés le ou les blocs B ioniques sont choisis parmi :
*Les monomères cationiques suivants :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges, les sels correspondants, ainsi que les macromonomères dérivant de tels monomères :
*Les monomères anioniques comprenant au moins une fonction carboxylique, sulfonique, sulfurique, phosphonique, phosphorique, sulfosuccinique, les sels correspondants, ainsi que les macromonomères dérivant de tels monomères.

11. Suspension selon l'une des revendications 1 à 10, **caractérisées en ce que** le rapport des masses molaires théoriques en poids, blocs A / blocs B est de préférence supérieur ou égal à 2.

12. Suspension selon l'une des revendications précédentes, **caractérisées en ce que** la quantité de polymère ioniquement chargé est comprise entre 0,01 et 30 % en poids par rapport à la phase aqueuse, plus particulièrement entre 0,05 et 10 % en poids ; de préférence entre 0,05 et 2 % en poids.

13. Suspension selon l'une des revendications précédentes, **caractérisées en ce que** la quantité de tensioactif est telle que le rapport Zs, correspondant à la concentration molaire de charge du tensioactif divisée par la concentration molaire en charge du polymère ioniquement chargé, est compris entre 0,01 et 100, plus particulièrement entre 0,1 et 10, de préférence entre 1 et 5.

14. Suspension selon l'une des revendications précédentes, **caractérisées en ce que** la quantité de copolymère à blocs, s'il est de même charge que le polymère ioniquement chargé, est telle que le rapport Zb, correspondant à la concentration molaire de charge du tensioactif divisée par la concentration molaire en charge du polymère ioniquement chargé et du copolymère à blocs, est compris entre 0,5 et 1,5 ; plus particulièrement entre 0,8 et 1,2.

15. Suspension selon l'une des revendications précédentes, **caractérisées en ce que** la quantité de copolymère à blocs, s'il est de charge opposée à celle du polymère ioniquement chargé, est telle que le rapport Z'b, correspondant à la concentration molaire de charge du tensioactif et du copolymère à blocs, divisée par la concentration molaire en charge du polymère ioniquement chargé, est compris entre 0,5 et 1,5 ; plus particulièrement entre 0,8 et 1,2.

16. Suspension selon l'une des revendications précédentes, **caractérisées en ce que** la quantité de copolymère à blocs, s'il ne possède pas de charge ionique, est comprise entre 10 et 100% en poids par rapport au poids de polymère ioniquement chargé et de tensioactif, le rapport Zs, correspondant à la concentration molaire de charge du tensioactif divisée par la concentration molaire en charge du polymère ioniquement chargé, étant compris entre 0,5 et 1,5 ; plus particulièrement entre 0,8 et 1,2.

17. Suspension selon l'une des revendications précédentes, **caractérisées en ce que** la matière active est choisie parmi les composés peu ou non miscibles à l'eau, se présentant sous une forme solide, liquide ou solubilisée dans un solvant non miscible à l'eau.

18. Suspension selon l'une des revendications précédentes, **caractérisées en ce que** la quantité de matière active est comprise entre 0,01 et 100% en poids par rapport au poids de polymère ioniquement chargé, de tensioactif et de copolymère à blocs.

19. Suspension selon l'une des revendications précédentes, **caractérisées en ce que** la concentration en particules est comprise entre 0,1 et 50 % en poids par rapport la phase aqueuse, de préférence entre 0,1 et 10 % en poids par rapport la phase aqueuse.

20. Procédé de préparation d'une suspension selon l'une des revendications 1 à 18, **caractérisé en ce que** l'on met en oeuvre les étapes suivantes
- On prépare un premier mélange aqueux comprenant le polymère ioniquement chargé et le cas échéant le copolymère à blocs si celui-ci est de même charge ;
- On prépare un deuxième mélange aqueux comprenant le tensioactif et le cas échéant, le copolymère à blocs si celui-ci est de même charge que le tensioactif ou s'il ne possède pas de charge ionique ;
- On ajoute le deuxième mélange au premier.

21. Procédé selon la revendication précédente, **caractérisé en ce que** l'on introduit la matière active dans le deuxième mélange ou une fois réalisé l'ajout du deuxième mélange au premier.

22. Utilisation de la suspension de particules décrite dans l'une des revendications 1 à 18, ou susceptible d'être obtenue selon le procédé décrit dans l'une des revendications 20 ou 21, comme agent modificateur d'état de surface.

23. Utilisation de la suspension de particules décrite dans l'une des revendications 1 à 18, ou susceptible d'être obtenue selon le procédé décrit dans l'une des revendications 20 ou 21, comme agent vectorisant de matières actives.

24. Utilisation de la suspension de particules décrite dans l'une des revendications 1 à 18, ou susceptibles d'être obtenue selon le procédé décrit dans l'une des revendications 20 ou 21, dans des formulations destinées au traitement de la peau et/ou du cheveu.

25. Utilisation de la suspension de particules décrite dans l'une des revendications 1 à 18, ou susceptible d'être obtenue selon le procédé décrit dans l'une des revendications 20 ou 21, dans des formulations destinées au traitement ou à la déformation de métaux.

26. Formulation destinée au traitement de la peau et/ou du cheveu, comprenant la suspension de particules décrite dans l'une des revendications 1 à 18, ou susceptible d'être obtenue selon le procédé décrit dans l'une des revendications 20 ou 21.

27. Formulation destinée au traitement et/ou à la déformation de métaux, comprenant la suspension de particules décrite dans l'une des revendications 1 à 18, ou susceptible d'être obtenue selon le procédé décrit dans l'une des revendications 20 ou 21

28. Utilisation de copolymères à blocs hydrosoluble possédant au moins un bloc A hydrophile non ionique et au moins un bloc B ionique ou hydrophobe non ionique comme agent stabilisant de particules dont la structure interne forme une mésophase et comprenant au moins un polymère hydrosoluble ioniquement chargé, au moins un tensioactif de charge ionique opposée à celle du polymère ; la structure interne des particules résultantes, qui se trouvent en suspension dans une phase aqueuse et comprennent le polymère ioniquement chargé, le tensioactif et le copolymère à blocs, formant la même mésophase.

29. Utilisation selon la revendication précédente, **caractérisée en ce que** les particules résultantes comprennent éventuellement au moins une matière active.

## Claims

1. Suspension in an aqueous phase of particles based on at least one ionically charged water-soluble polymer, at least one surfactant having an ionic charge opposite to that of said polymer, at least one water-soluble block copolymer possessing at least one nonionic hydrophilic block A and at least one nonionic hydrophobic or ionic block B, the particles optionally comprising at least one active substance, the internal structure of the particles forming a mesophase.

2. Suspension as claimed in one of the preceding claims, **characterized in that** the ionically charged polymer is a homopolymer.

3. Suspension as claimed in one of the preceding claims, **characterized in that** the ionically charged polymer is a random copolymer.

4. Suspension as claimed in the preceding claim, **characterized in that** the ionically charged polymer is obtained from monomers which carry the same type of ionic charge and of which at least 80 mol% is ionically charged under the pH conditions of the composition and of the suspension.

5. Suspension as claimed in one of the preceding claims, **characterized in that** the ionically charged polymer is obtained from nonionic hydrophilic monomers or nonionic hydrophobic monomers; the molar percentage of monomers of this type in the polymer is less than or equal to 30%, preferably less than or equal to 10%.

6. Suspension as claimed in one of the preceding claims, **characterized in that** the surfactant is selected from anionic or cationic surfactants and **in that** it may comprise one or more nonionic surfactants in a proportion of less than 30 mol% relative to the ionic surfactant, preferably less than 10 mol%.

7. Suspension as claimed in one of the preceding claims, **characterized in that** the ionically charged polymer and the surfactant form a mesophase under the same concentration conditions as those of the particles as claimed in the invention.

8. Suspension as claimed in one of the preceding claims, **characterized in that** the monomers from which the block or blocks A are formed are selected from ethylene oxide; amides of monocarboxylic or polycarboxylic acids, linear, branched, cyclic or aromatic, containing at least one ethylenic unsaturation or derivatives thereof; hydrophilic esters deriving from (meth)acrylic acid; vinyl esters which allow polyvinyl alcohol blocks to be obtained after hydrolysis; and monomers of the sugars type; alone or in mixtures, and also macromonomers deriving from such monomers.

9. Suspension as claimed in one of the preceding claims, **characterized in that** the monomers from which the nonionic hydrophobic block or blocks B are formed are selected from:
- esters of monocarboxylic or polycarboxylic acids, linear, branched, cyclic or aromatic, containing at least one ethylenic unsaturation;
- α,β-ethylenically unsaturated nitriles, vinyl ethers, vinyl esters, vinylaromatic monomers and vinyl or vinylidene halides;
- linear or branched, aromatic or nonaromatic hydrocarbon monomers containing at least one ethylenic unsaturation;
alone or in mixtures, and also macromonomers deriving from such monomers.

10. Suspension as claimed in one of claims 1 to 8, **characterized in that** the monomers from which the ionic block or blocks B are formed are selected from:
*the following cationic monomers:
- aminoalkyl (meth)acrylates and aminoalkyl(meth)acrylamides;
- monomers containing at least one secondary, tertiary or quaternary amine function, or a heterocyclic group containing a nitrogen atom, vinylamine and ethyleneimine;
- diallyldialkylammonium salts;
alone or in mixtures, the corresponding salts, and macromonomers deriving from such monomers;
*anionic monomers containing at least one carboxylic, sulfonic, sulfuric, phosphonic, phosphoric or sulfosuccinic function, the corresponding salts, and macromonomers deriving from such monomers.

11. Suspension as claimed in one of claims 1 to 10, **characterized in that** the ratio of the theoretical molar masses by weight of blocks A/blocks B is preferably greater than or equal to 2.

12. Suspension as claimed in one of the preceding claims, **characterized in that** the amount of ionically charged polymer is between 0:01 and 30% by weight relative to the aqueous phase, more particularly between 0.05 and 10% by weight, preferably between 0.05 and 2% by weight.

13. Suspension as claimed in one of the preceding claims, **characterized in that** the amount of surfactant is such that the ratio Zs, corresponding to the molar charge concentration of the surfactant divided by the molar charge concentration of the ionically charged polymer, is between 0.01 and 100, more particularly between 0.1 and 10, preferably between 1 and 5.

14. Suspension as claimed in one of the preceding claims, **characterized in that** the amount of block copolymer, if it has the same charge as the ionically charged polymer, is such that the ratio Zb, corresponding to the molar charge concentration of the surfactant divided by the molar charge concentration of the ionically charged polymer and of the block copolymer, is between 0.5 and 1.5, more particularly between 0.8 and 1.2.

15. Suspension as claimed in one of the preceding claims, **characterized in that** the amount of block copolymer, if it has a charge opposite to that of the ionically charged polymer, is such that the ratio Z'b, corresponding to the molar charge concentration of the surfactant and of the block copolymer divided by the molar charge concentration of the ionically charged polymer, is between 0.5 and 1.5, more particularly between 0.8 and 1.2

16. Suspension as claimed in one of the preceding claims, **characterized in that** the amount of block copolymer, if it has no ionic charge, is between 10 and 100% by weight relative to the weight of ionically charged polymer and of surfactant, the ratio Zs, corresponding to the molar charge concentration of the surfactant divided by the molar charge concentration of the ionically charged polymer, being between 0.5 and 1.5, more particularly between 0.8 and 1.2.

17. Suspension as claimed in one of the preceding claims, **characterized in that** the active substance is selected from compounds with low or no miscibility with water which are in solid or liquid form or in dissolved form in a water-immiscible solvent.

18. Suspension as claimed in one of the preceding claims, **characterized in that** the amount of active substance is between 0.01 and 100% by weight relative to the weight of ionically charged polymer, surfactant and block copolymer.

19. Suspension as claimed in one of the preceding claims, **characterized in that** the concentration of particles is between 0.1 and 50% by weight relative the aqueous phase, preferably between 0.1 and 10% by weight relative the aqueous phase.

20. A process for preparing a suspension as claimed in one of claims 1 to 18, **characterized in that** the following steps are implemented:
- a first aqueous mixture is prepared, comprising the ionically charged polymer and where appropriate the block copolymer if its charge is the same;
- a second aqueous mixture is prepared, comprising the surfactant and where appropriate the block copolymer if its charge is the same as the surfactant or if it has no ionic charge;
- the second mixture is added to the first.

21. The process as claimed in the preceding claim, **characterized in that** the active substance is introduced into the second mixture or once the addition of the second mixture to the first has been made.

22. The use of the suspension of particles described in one of claims 1 to 18 or obtainable by the process described in one of claims 20 or 21 as a surface state modifier.

23. The use of the suspension of particles described in one of claims 1 to 18 or obtainable by the process described in one of claims 20 or 21 as an active substance carrier.

24. The use of the suspension of particles described in one of claims 1 to 18 or obtainable by the process described in one of claims 20 or 21 in formulations intended for treating the skin and/or hair.

25. The use of the suspension of particles described in one of claims 1 to 18 or obtainable by the process described in one of claims 20 or 21 in formulations intended for treating or forming metals.

26. A formulation intended for treating the skin and/or hair, comprising the suspension of particles described in one of claims 1 to 18 or obtainable by the process described in one of claims 20 or 21.

27. A formulation intended for treating and/or shaping metals, comprising the suspension of particles described in one of claims 1 to 18 or obtainable by the process described in one of claims 20 or 21.

28. The use of water-soluble block copolymers possessing at least one nonionic hydrophilic block A and at least one nonionic hydrophobic or ionic block B as a stabilizer of particles whose internal structure forms a mesophase and which comprise at least one ionically charged water-soluble polymer and at least one surfactant having an ionic charge opposite to that of the polymer; the internal structure of the resultant particles, which are in suspension in an aqueous phase and comprise the ionically charged polymer, the surfactant and the block copolymer, forming the same mesophase.

29. The use as claimed in the preceding claim, **characterized in that** the resultant particles optionally comprise at least one active substance.

## Patentansprüche

1. Suspension in wässriger Phase von Partikeln auf Basis wenigstens eines wasserlöslichen, ionisch geladenen Polymers, wenigstens eines Tensids mit entgegengesetzt ionischer Ladung zu jener des Polymers, wenigstens eines wasserlöslichen Blockcopolymers, das wenigstens einen hydrophilen nicht ionischen Block A und wenigstens einen ionischen oder hydrophoben nicht ionischen Block B aufweist, wobei die Partikel gegebenenfalls wenigstens einen Wirkstoff umfassen; wobei die interne Struktur der Partikel eine Mesophase bildet.

2. Suspension gemäß einem der vorausgegangenen Ansprüche, **dadurch gekennzeichnet, dass** das ionisch geladene Polymer ein Homopolymer ist.

3. Suspension gemäß einem der vorausgegangenen Ansprüche, **dadurch gekennzeichnet, dass** das ionisch geladene Polymer ein statistisches Copolymer ist.

4. Suspension gemäß dem vorausgegangenen Anspruch, **dadurch gekennzeichnet, dass** das ionisch geladene Polymer aus Monomeren erhalten wird, die die gleiche Art ionischer Ladung tragen und von denen wenigstens 80 Mol-% unter pH-Bedingungen der Zusammensetzung und der Suspension ionisch geladen sind.

5. Suspension gemäß einem der vorausgegangenen Ansprüche, **dadurch gekennzeichnet, dass** das ionisch geladene Polymer aus nicht ionischen hydrophilen oder nicht ionischen hydrophoben Monomeren erhalten wird; wobei der Mol-Prozentsatz dieser Art Monomere in dem Polymer kleiner oder gleich 30%, vorzugsweise kleiner oder gleich 10% ist.

6. Suspension gemäß einem der vorausgegangenen Ansprüche, **dadurch gekennzeichnet, dass** das Tensid aus den anionischen oder kationischen Tensiden gewählt ist, und **dadurch**, dass sie ein oder mehrere nicht ionische Tenside mit einem Gehalt von weniger als 30 Mol-% bezogen auf das ionische Tensid, vorzugsweise weniger als 10 Mol-%, aufweisen kann.

7. Suspension gemäß einem der vorausgegangenen Ansprüche, **dadurch gekennzeichnet, dass** das ionisch geladene Polymer und das Tensid eine Mesophase bilden, unter den gleichen Konzentationsbedingungen wie die Partikel gemäß der Erfindung.

8. Suspension gemäß einem der vorausgegangenen Ansprüche, **dadurch gekennzeichnet, dass** die Monomere, aus denen der oder die Blöcke A gebildet sind, gewählt sind aus Ethylenoxid; den Amiden der Mono- oder Polycarbonsäuren, linear, verzweigt, zyklisch oder aromatisch, die wenigstens einfach ungesättigt sind oder deren Derivate umfassen; hydrophilen Estern, die von (Meth)acrylsäure abstammen; Vinylestern, die es ermöglichen, nach Hydrolyse Polyvinylalkoholblöcke zu erhalten; Monomeren der Art Zucker;
einzeln oder als Gemisch, sowie den Makromonomeren, die von diesen Monomeren abstammen.

9. Suspension gemäß einem der vorausgegangenen Ansprüche, **dadurch gekennzeichnet, dass** die Monomere, aus denen der oder die hydrophoben nicht ionischen Blöcke B gebildet sind, gewählt sind aus:
- den Estern der Mono- oder Polycarbonsäuren, linear, verzweigt, zyklisch oder aromatisch, die wenigstens einfach ungesättigt sind;
- den αβ-ethylenisch ungesättigten Nitrilen, Vinylethern, Vinylestern, vinylaromatischen Monomeren, Vinyl- oder Vinylidenhalogeniden;
- den Kohlenwasserstoffmonomeren, linear oder verzweigt, aromatisch oder nicht, die wenigstens einfach ungesättigt sind;
einzeln oder als Gemisch, sowie den Makromonomeren, die von diesen Monomeren abstammen.

10. Suspension gemäß einem der Ansprüche 1 bis 8, **dadurch gekennzeichnet, dass** die Monomere, aus denen der oder die ionischen Blöcke B gebildet sind, gewählt sind aus:
* den folgenden kationischen Monomeren:
- Aminoalkyl(meth)acrylate, Aminoalkyl(meth)acrylamide;
- Monomere mit wenigstens einer sekundären, tertiären oder quaternären Aminfunktion, oder eine heterozyklische Gruppe, die ein Stickstoffatom enthält, Vinylamin, Ethylenimin;
- Diallyldialkylammoniumsalze;
einzeln oder als Gemisch, den entsprechenden Salzen sowie den Makromonomeren, die von diesen Monomeren abstammen;
* den anionischen Monomeren mit wenigstens einer Carboxyl-, Sulfon-, Schwefel-, Phosphon-, Phosphor-, Sulfobernsteinfunktion, den entsprechenden Salzen sowie den Makromonomeren, die von diesen Monomeren abstammen.

11. Suspension gemäß einem der Ansprüche 1 bis 10, **dadurch gekennzeichnet, dass** das Verhältnis theoretischer Molmassen in Gewicht Blöcke A / Blöcke B vorzugsweise größer oder gleich 2 ist.

12. Suspension gemäß einem der vorausgegangenen Ansprüche, **dadurch gekennzeichnet, dass** die Menge des ionisch geladenen Polymers zwischen 0,01 und 30 Gew.-%, genauer zwischen 0,05 und 10 Gew.-%, vorzugsweise zwischen 0,05 und 2 Gew.-%, bezogen auf die wässrige Phase beträgt.

13. Suspension gemäß einem der vorausgegangenen Ansprüche, **dadurch gekennzeichnet, dass** die Menge an Tensid derart ist, dass das Verhältnis Zs, das der Molkonzentration der Ladung des Tensids geteilt durch die Molkonzentration bezogen auf die Ladung des ionisch geladenen Polymers entspricht, zwischen 0,01 und 100, genauer zwischen 0,1 und 10, vorzugsweise zwischen 1 und 5, liegt.

14. Suspension gemäß einem der vorausgegangenen Ansprüche, **dadurch gekennzeichnet, dass** die Menge des Blockcopolymers, wenn es die gleiche Ladung aufweist wie das ionisch geladene Polymer, derart ist, dass das Verhältnis Zb, das der Molkonzentration der Ladung des Tensids geteilt durch die Molkonzentration bezogen auf die Ladung des ionisch geladenen Polymers und des Blockcopolymers entspricht, zwischen 0,5 und 1,5, genauer zwischen 0,8 und 1,2, liegt.

15. Suspension gemäß einem der vorausgegangenen Ansprüche, **dadurch gekennzeichnet, dass** die Menge des Blockcopolymers, wenn es die zum ionisch geladenen Polymer entgegengesetzte Ladung aufweist, derart ist, dass das Verhältnis Z'b, das der Molkonzentration der Ladung des Tensids und des Blockcopolymers geteilt durch die Molkonzentration bezogen auf die Ladung des ionisch geladenen Polymers entspricht, zwischen 0,5 und 1,5, genauer zwischen 0,8 und 1,2, liegt.

16. Suspension gemäß einem der vorausgegangenen Ansprüche, **dadurch gekennzeichnet, dass** die Menge an Blockcopolymer, wenn es nicht ionisch geladen ist, zwischen 10 und 100 Gew.-% bezogen auf das Gewicht des ionisch geladenen Polymers und des Tensids liegt, wobei das Verhältnis Zs, das der Molkonzentration der Ladung des Tensids geteilt durch die Molkonzentration bezogen auf die Ladung des ionisch geladenen Polymers entspricht, zwischen 0,5 und 1,5, genauer zwischen 0,8 und 1,2, liegt.

17. Suspension gemäß einem der vorausgegangenen Ansprüche, **dadurch gekennzeichnet, dass** der Wirkstoff aus den Verbindungen gewählt ist, die wenig oder nicht mit Wasser mischbar sind und in fester, in flüssiger oder in einem nicht mit Wasser mischbaren Lösungsmittel gelöster Form vorliegen.

18. Suspension gemäß einem der vorausgegangenen Ansprüche, **dadurch gekennzeichnet, dass** die Menge an Wirkstoff zwischen 0,01 und 100 Gew.-% bezogen auf das Gewicht des ionisch geladenen Polymers, des Tensids und des Blockcopolymers beträgt.

19. Suspension gemäß einem der vorausgegangenen Ansprüche, **dadurch gekennzeichnet, dass** die Konzentration an Partikeln zwischen 0,1 und 50 Gew.-% bezogen auf die wässrige Phase, vorzugsweise zwischen 0,1 und 10 Gew.-% bezogen auf die wässrige Phase, beträgt.

20. Verfahren zur Herstellung einer Suspension gemäß einem der Ansprüche 1 bis 18, **dadurch gekennzeichnet, dass** die folgenden Schritte durchgeführt werden:
- es wird ein erstes wässriges Gemisch zubereitet, das das ionisch geladene Polymer und gegebenenfalls das Blockcopolymer, sofern dieses die gleiche Ladung aufweist, umfasst;
- es wird ein zweites wässriges Gemisch zubereitet, das das Tensid und gegebenenfalls das Blockcopolymer, sofern dieses die gleiche Ladung wie das Tensid aufweist oder gar nicht ionisch geladen ist, umfasst;
- das zweite Gemisch wird zu dem ersten hinzugefügt.

21. Verfahren gemäß dem vorausgegangenen Anspruch, **dadurch gekennzeichnet, dass** der Wirkstoff in das zweite Gemisch eingebracht wird oder, sobald durchgeführt, das zweite Gemischs zum ersten hinzugefügt wird.

22. Verwendung der Suspension aus Partikeln wie in einem der Ansprüche 1 bis 18 beschrieben oder geeignet, gemäß dem in einem der Ansprüche 20 oder 21 beschriebenen Verfahren erhalten zu werden, als Mittel zur Modifizierung der Oberflächenbeschaffenheit.

23. Verwendung der Suspension aus Partikeln wie in einem der Ansprüche 1 bis 18 beschrieben oder geeignet, gemäß dem in einem der Ansprüche 20 oder 21 beschriebenen Verfahren erhalten zu werden, als Mittel zur Vektorisierung der Wirkstoffe.

24. Verwendung der Suspension aus Partikeln wie in einem der Ansprüche 1 bis 18 beschrieben oder geeignet, gemäß dem in einem der Ansprüche 20 oder 21 beschriebenen Verfahren erhalten zu werden, in Formulierungen zur Behandlung der Haut und/oder des Haars.

25. Verwendung der Suspension aus Partikeln wie in einem der Ansprüche 1 bis 18 beschrieben oder geeignet, gemäß dem in einem der Ansprüche 20 oder 21 beschriebenen Verfahren erhalten zu werden, in Formulierungen zur Behandlung oder Verformung von Metallen.

26. Formulierung zur Behandlung der Haut und/oder des Haars, welche die Suspension aus Partikeln wie in einem der Ansprüche 1 bis 18 beschrieben oder geeignet, gemäß dem in einem der Ansprüche 20 oder 21 beschriebenen Verfahren erhalten zu werden, umfasst.

27. Formulierung zur Behandlung und/oder Verformung von Metallen, welche die Suspension aus Partikeln wie in einem der Ansprüche 1 bis 18 beschrieben oder geeignet, gemäß dem in einem der Ansprüche 20 oder 21 beschriebenen Verfahren erhalten zu werden, umfasst.

28. Verwendung von wasserlöslichen Blockcopolymeren, die wenigstens einen hydrophilen nicht ionischen Block A und wenigstens einen ionischen oder hydrophoben nicht ionischen Block B aufweisen, als Mittel zur Stabilisierung von Partikeln, deren interne Struktur eine Mesophase bildet, und die wenigstens ein wasserlösliches ionisch geladenes Polymer, wenigstens ein Tensid mit entgegengesetzt ionischer Ladung zu jener des Polymers aufweisen; wobei die interne Struktur der sich ergebenden Partikel, die sich in Suspension in einer wässrigen Phase befinden und das ionisch geladene Polymer umfassen, das Tensid und das Blockcopolymer die gleiche Mesophase bilden.

29. Verwendung gemäß dem vorausgegangenen Anspruch, **dadurch gekennzeichnet, dass** die sich ergebenden Partikel gegebenenfalls wenigstens einen Wirkstoff umfassen.
